# EUROPEAN PATENT SPECIFICATION

(11) **EP 1 572 315 B1**
(45) Date of publication and mention of the grant of the patent: **26.11.2008**
(21) Application number: 03767821.6
(22) Date of filing: 18.12.2003
(51) Int. Cl.: B01D 11/04, A61K 9/14

(54) **APPARATUS AND METHOD FOR THE ISOLATION OF PRODUCED PARTICLES AS A SUSPENSION IN A NON-SUPERCRITICAL FLUID**
VORRICHTUNG UND VERFAHREN ZUR TRENNUNG VON ERZEUGTEN PARTIKELN ALS SUSPENSION IN EINEM NICHT SUPERKRITISCHEN FLUID
APPAREIL ET PROCEDE D'ISOLATION DE PARTICULES PRODUITES, EN SUSPENSION DANS UN FLUIDE NON SUPERCRITIQUE

(30) Priority: 20.12.2002 GB 0229714
(43) Date of publication of application: 14.09.2005
(60) Divisional application: 08160038.9
(73) Proprietor: GLAXO GROUP LIMITED, Greenford, Middlesex UB6 ONN (GB)
(72) Inventor: COOPER, Simon, Murray, GlaxoSmithKline, Ware, Hertfordshire SG12 0DP (GB); MERRIFIELD, David, Roy, GlaxoSmithKline, Harlow, Essex CM19 5AW (GB); ROBERTSON, John, GlaxoSmithKline, Harlow, Essex CM19 5AW (GB); VALDER, Christopher, Edmund, GlaxoSmithKline, Harlow, Essex CM19 5AW (GB)
(74) Representative: Povey, Alexander W.G.
(86) International application number: PCT/EP2003/014550
(87) International publication number: WO 2004/056443

(56) References cited:
- EP-A- 1 005 903
- WO-A-99/65469
- US-A- 5 252 224
- US-A- 5 543 057
- US-A1- 2002 179 540
- US-B1- 6 440 337

## Description

This invention relates to a process for the isolation of particulate products from high pressure, i.e. supercritical, environments.

The use of supercritical fluids (SCF's) and the properties thereof have been extensively documented, see for example, McHugh M.A. and Krukonis V.J., Supercritical Fluid Extraction: Principles and Practice, Butterworth-Heinemann, 2nd Ed., 1994*.* King M.B. and Bott T.R., Extraction of Natural Products Using Near Critical Solvents, Blackie Academic and Professional, Glasgow, 1993 *and* Krukonis V.J., Brunner G. and Perrut M., Industrial Operations with Supercritical Fluids: Current Processes and Perspectives on the Future, Tome 1, Proceedings of the 3rd Int. Symp. on Supercritical Fluids, 1994.

A supercritical fluid is a fluid which is above both its critical pressure (Pc) and critical temperature (Tc). Supercritical fluids are of considerable interest in a number of fields of endeavour because of their unique properties. These properties include self diffusivities and viscosities approaching that of a gas, densities approaching that of a liquid and zero surface tension. Furthermore, the high compressibility of supercritical fluids implies large changes in fluid density for small changes in pressure, which in turn results in highly controllable solvation power and thus selective extraction using a single supercritical fluid is possible. Furthermore, many supercritical fluids are gases at ambient conditions of temperature and pressure, which eliminates the evaporation or concentration step needed in conventional liquid extraction. The densities of supercritical fluids typically range from 0.1-1.4 gml⁻¹ under normal working conditions.

Most of the commonly used supercritical fluids provide advantageous environments for working with compounds of low stability due to their inertness and the moderate temperatures used in routine working conditions. Carbon dioxide is the most extensively used SCF as it is cheap, readily available, inert, non-toxic, non flammable, and has a critical temperature near to ambient temperature.

The unique characteristics of SCF's have led to the development of many techniques in the fields of extraction, crystallisation, precipitation, reaction chemistry, polymer chemistry, and analytical science.

The SCF techniques of most relevance to the present invention are those employed in the fields of crystallisation, precipitation, and the processing of solids. Several methods and techniques have been developed in the field of supercritical fluid crystallisation, precipitation, and solids processing. In general these methods are designed to produce a finely divided solid product. These methods generally fall into three classes as follows:
1). Methods wherein the supercritical fluid is used as a solvent. Techniques such as the Rapid Expansion of Supercritical fluids (RESS) fall into this class.
2). Methods wherein the supercritical fluid is used as an anti-solvent or nonsolvent. Techniques such as Gas-Anti Solvent (GAS), Supercritical Anti-Solvent (SAS), Precipitation with a Compressed fluid Anti-solvent (PCA), Aerosolised Supercritical Extraction System (ASES), and Solution Enhanced Dispersion of Solids (SEDS) are examples which fall within this class.
3). Methods wherein the supercritical fluid is used as a solute and dispersing aid. The technique Particles from Gas Saturated Solutions (PGSS) is an example which falls within this area.

All of the aforementioned SCF particle formation methods are documented in the scientific literature and thus familiar to those skilled in the art. In cases where the SCF is used as a solvent, RESS is generally applied (see, for example, Mohamed R.S., Halverson D. S., Debenedetti P. G. and Prud'homme R.K., Solids Formation after the Expansion of Supercritical Mixtures, Chapt 23 in A.C.S. Supercritical Science and Technology, 1989). This method generally involves the dissolution of the solute of interest in a supercritical fluid, followed by rapid expansion of the supercritical solution to a lower pressure, for example atmospheric pressure, resulting in the precipitation of particles of the solute. In cases where the supercritical fluid is used as an anti-solvent, the GAS recrystallisation or evolved versions of it (such as SEDS, PCA, SAS or ASES) are generally applied. These techniques are all described in the scientific literature, see for example P.M. Gallagher et al, Supercritical Fluid Science and Technology, ACS Symp. Ser., 406, p.334 (1989*) and* Dixon D.J., Johnston K.P. and Bodmeier R.A., Polymeric Materials formed by Precipitation with a Compressed Fluid Anti-Solvent, J.AIChE., Vol. 39, No. 1, 1993, pp127-139). In the GAS technique, the solute of interest is dissolved in a conventional solvent. A supercritical fluid such as carbon dioxide is then introduced into or mixed with the solution which dissipates into the supercritical fluid (and vice versa). As a result of this dissipation of the molecules of the solution, a supersaturation with respect to the formation of a solid phase of a component of interest may be brought about. It is recognised within the field that the rate of attainment and degree of supersaturation in processes such as supercritical fluid processes can be altered over many orders of magnitude. It is also recognised that this manipulation may be brought about by appropriate adjustment and control of some or all of the numerous process variables. It is further recognised that such manipulations can have an advantageous impact upon the physicochemical properties of divided materials.

In cases where the supercritical fluid is used as a solute, such as in the PGSS process, the supercritical fluid is dissolved in a melt or dispersion of the material of interest. Upon depressurisation, the supercritical fluid expands to a gas. The resulting increase in volume of the SCF causes the material in which it was dissolved to atomise into small droplets or subunits. It is from these droplets that the solid product is the formed (see for example Weider, E., Steiner R. and Knez Z., "Powder Generation from Polyethyleneglycols with Compressible Fluids", High Pressure Chemical Engineering, Ph. Rudolph von Rohr and Ch. Trepp (Eds.), Elsevier Science B. V., 1996, pp 223-228*)*.

All of these techniques, when applied to particle formation, have their limitations and serious practical difficulties associated with the handling of particulate materials being fed to or produced from supercritical processes. These difficulties arise for a number of reasons including low particle size which leads to a cohesive, non-free flowing product. The high pressures under which the processes operate and the cumbersome design and construction of the valves, vessels, and pipework of the high pressure system make the equipment difficult to operate and access to the product recovery areas of the equipment becomes increasingly restricted as these processes are scaled up. These difficulties have been recognised and some efforts have been made to address them. For example, International Patent Application publication number WO 01/43845 (Separex) discloses a method for capturing very fine particles present in a fluid flux in liquid, gaseous, or supercritical state by trapping the particles within a solid carbon dioxide mixture. WO 01/43853 A1 (Separex (Société Anonyme)) describes a process which captures the very finely divided solid produced from an SCF process by trapping the small particles on a bed of larger granule. US 2002/01799540 discloses the isolation of time particles as a dry powder from carbon dioxide show.

It has now surprisingly been found that the problems associated with the recovery, handling, and transport of the product may be overcome by isolation of the particulates as a suspension in a non-supercritical liquefied gas. Particular advantages of this method include the avoidance of handling a cohesive, non-free flowing solid material and its associated problems. A further advantage is that by using this method, finely divided or divided materials may readily be transferred to high pressure, for example, supercritical processes such as RESS and PGSS processes. Furthermore, the solid product may readily be obtained by displacement or volatilisation of the non-supercritical liquid and in cases where a non-volatile, soluble component is included in the fluid, it may be deposited with the particulates. A still further advantage of this process is that inclusion or suspension of material(s) in the non-supercritical liquefied gas allows two or more materials to be transported, processed through a unit operation (for example mixed) and isolated as a powder product. Few modifications to the high pressure system are necessary in order to incorporate the apparatus and smaller high-pressure vessels may be used thus offering significant cost savings.

Accordingly, there is provided a process for the isolation of the product from a supercritical pressure process which process comprises the isolation of the product as a suspension in a non-supercritical fluid. The process of the invention is defined in claim 1.

It will be appreciated that the supercritical fluid and non-supercritical fluid may be composed of the same fluid or the supercritical fluid and non-supercritical fluid may be composed of different fluids.

It will further be appreciated that the process for the isolation of the product from a high pressure, for example supercritical, process, is equally applicable to the isolation of a product which product comprises more than one component wherein each component has been formed in separate, high pressure, for example supercritical, processes. An example of a product comprising more than one component is a product wherein one component forms a partial or complete coat on another component. Suitably, a product comprising more than one component is a product comprising two components.

Described herein is an apparatus for the isolation of the product of a supercritical fluid process. A process in which the supercritical fluid is used as an anti-solvent for the formation of a particulate product will be used to exemplify the application of the invention and the advantages conferred by the invention on the handling and transport of the product. It should be noted that by suitable rearrangement of the said apparatus, the advantages conferred on the handling and transport of the product can be applied to other processes which require the transfer of finely divided solids either to or from a high pressure, for example supercritical, environment.

The apparatus comprises one or more collection vessels with a means of controlling the temperature and pressure of said vessels, and a means for the introduction of a non-supercritical fluid into one or more particle formation vessels, and optionally a homogenisation vessel. The particle formation vessel is connected to the collection vessel, optionally by way of a homogenisation vessel, which homogenisation vessel is provided with means for agitating the contents of the said vessel and optionally a means for recirculating the contents of and within the said homogenisation vessel.

The apparatus may comprise a means for the introduction of a non-supercritical fluid into one or more particle formation vessels, one or more collection vessels with a means of controlling the temperature and pressure of said collection vessels, and optionally a homogenisation vessel located between the particle formation vessel(s) and the collection vessel(s).

The product is provided as a suspension which may consist of one or more components which have been prepared by a particle formation process as described, with one or more components that have been prepared in a different way, and introduced into the described process by charging as powders.

In addition the process as described in the claims facilitates continuous or semi-continuous formation and isolation of products from a supercritical pressure process.

It will be appreciated that the means for the introduction of a non-supercritical fluid may be connected to more than one particle formation vessel and that more than one collection vessel may be used. It will further be appreciated that the incorporation of a homogenisation vessel is advantageous if more than one particle formation vessel is used. Furthermore, it will also be appreciated that the incorporation of the homogenisation step is advantageous if the process is used to produce a material made up of multiple components which have been formed by the particle step or have been added to the homogenisation vessel by other or similar means. The homogenisation step may also be advantageously incorporated into other high pressure, for example supercritical, processes such as RESS.

Accordingly, apparatus may comprise a stirred vessel or vessels to contain the components in suspension and a homogeniser such as a rotor stator homogeniser.

The process may comprise the steps of mixing the particulate components with the non-supercritical fluid, stirring the particulate components in the non-supercritical fluid to create a slurry or suspension and a step whereby this slurry of suspension is fed through a homogeniser and returned to the bulk suspension.

It will also be appreciated that the use of more than one particle formation vessel and/or more than one collection vessel may allow for essentially continuous operation of the process by switching from one particle formation vessel to another and/or switching from one collection vessel to another, thus providing the advantages offered by continuous operation such as reduced cost and improved safety. It will be understood that the particle formation vessel is part of the apparatus for the formation of the particulate product.
In a preferred embodiment, the means for the introduction of a non-supercritical fluid is connected to two particle formation vessels, which in turn are connected to a homogenisation vessel, which homogenisation vessel is connected to a particle collection vessel.

Suitable high pressure processes in which the isolation process of the present invention may be utilised include processes utilising supercritical fluids. Examples of suitable supercritical fluid processes include, but are not limited to, RESS, GAS, PGSS, SEDS, SAS, PCA and ASES, especially GAS and PCA.

As used herein, the term "supercritical fluid" means a fluid which is simultaneously at or above its critical pressure (Pc) and critical temperature (Tc).

Suitably, the pressure of the fluid is likely to be in the range 1.01 Pc - 7.0 Pc, and its temperature in the range 1.01 Tc - 4.0 Tc.

Suitable fluids for use in the process include nitrous oxide, sulphur hexafluoride, xenon, ethylene, chlorotrifluoromethane, ethane, trifluoromethane, 1,1,1,2-tetrafluoroethane and carbon dioxide.

A preferred fluid for use in the process is carbon dioxide.

The supercritical fluid may optionally contain one or more modifiers, for example, but not limited to, methanol, ethanol, iso-propyl alcohol or acetone. When used, the modifier preferably constitutes not more than 20%, and more particularly constitutes between 1 and 10%, of the supercritical fluid.

The term "modifier" is well known to those persons skilled in the art. A modifier (or co-solvent) may be described as a chemical which, when added to a supercritical fluid, changes the intrinsic properties of the supercritical fluid.

If will be appreciated that the choice of supercritical fluid and the concentration of any modifier will be dependent upon the particular product being handled. The choice of a suitable combination of supercritical fluid, and modifier (where desired) for any particular product will be well within the capabilities of a person of ordinary skill in the art.

The term "particulate product" includes products in a single-component or multi-component form. Examples of multi-component forms include intimate mixtures of one component in a matrix of another component. Particulate products which may be isolated using the present invention include pharmaceutical products. Examples of pharmaceutical products include pharmaceutically active compounds and pharmaceutical excipients. Suitable particulate products are pharmaceutically active compounds and excipients. Suitable pharmaceutically active products include fluticasone propionate, ropinirole hydrochloride, salmeterol xinafoate and paracetamol. A further suitable pharmaceutically active product is furan-2-carboxylic acid (6S,8S,9R,10S,11S,13S,14S,16R,17R)-6,9-difluoro-17-fluoromethylsulfanylcarbonyl-11-hydroxy-10,13,16-trimethyl-3-oxo-6,7,8,9,10,11,12,13,14,15,16,17-dodecahydro-3H-cyclopenta[a]phenanthren-17-ylester (hereinafter referred to as "Compound A"). Suitable excipients include lactose, sodium lauryl sulphate, hydroxymethylcellulose and polyethyleneglycols.

It will also be appreciated that the precise conditions of operation of the process of the invention will be dependent upon the choice of supercritical fluid and whether or not modifiers are present.

The critical pressures and critical temperatures for some selected fluids are listed in Table 1:

**Table 1**

| Fluid | Pc (bar) | Tc (°C) |
|---|---|---|
| Carbon dioxide | 74 | 31 |
| Nitrous oxide | 72 | 36 |
| Sulphur hexafluoride | 37 | 45 |
| Xenon | 58 | 16 |
| Ethylene | 51 | 10 |
| Chlorotrifluoromethane | 39 | 29 |
| 1,1,1,2 - tetrafluoroethane | 40 | 101 |
| Ethane | 48 | 32 |
| Trifluoromethane | 47 | 26 |

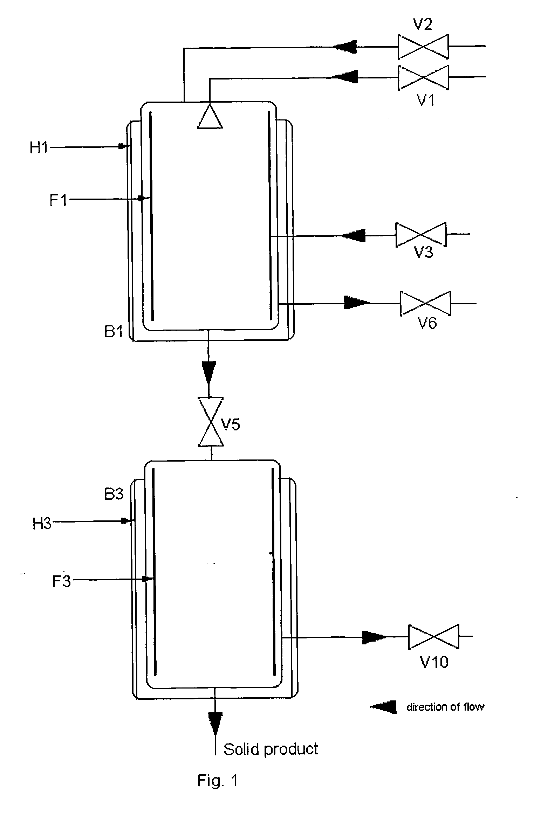

Fig. 1 is a simplified schematic diagram of an apparatus according one embodiment of the invention. The apparatus as illustrated in Fig. 1 consists of a particle formation vessel B1 of an appropriate capacity. Within this embodiment, the configuration is such to allow the application of the invention to cases where the supercritical fluid is used as an anti-solvent in processes such as PCA, SAS, SEDS etc. Such a configuration will be recognised as appropriate to those skilled in the art.

The temperature and pressure of the vessel are maintained at the desired values by means of a jacket heater (H1) and by controlling the temperature of the supercritical fluid feed-stream fed into the apparatus *via* valve V2 and the temperature of the solution feed stream fed into the apparatus *via* valve V1. Suitable fluids are those which form supercritical fluids, for example; nitrous oxide, sulphur hexafluoride, xenon, ethylene, chlorotrifluoromethane, ethane, trifluoromethane, tetrafluoroethane and carbon dioxide, which fluids may be used either with or without the addition of one or more suitable modifiers. The system is brought to operating temperature and a suitable fluid, for example supercritical carbon dioxide, is fed into B1 through valve V2, filtered as it passes through filter F1 and is allowed to exit vessel B1 via back-pressure regulator V6. A solution of the solid of interest in a suitable solvent, for example methanol, is then fed into B1 through valve V1. The solid of interest then begins to precipitate and is collected on radial filter F1. The operation of a particle formation vessel of a similar type is disclosed in numerous publications describing the use of supercritical fluids as anti-solvents e.g. Dixon D.J., Johnston K.P. and Bodmeier R.A., Polymeric Materials formed by Precipitation with a Compressed Fluid Anti-Solvent, J.AIChE., Vol. 39, No.1, 1993, pp127-139*)*. When sufficient precipitated material has been collected, V1 is closed and the flow of solution to the system stopped. It is recognised that in some cases, flushing of the solution feed line with pure solvent may be necessary to displace residual solution. Flow of pure supercritical fluid through the system is maintained for a sufficient length of time so as to flush vessel B1 free of any SCF-solvent mixture. The time required to flush may be determined readily by those skilled in the art. Generally within the present embodiment of the invention this flushing time corresponds to, but is not limited to, the time taken to displace three vessel volumes. Valve V2 is then closed and vessel B1 partially depressurised to below the critical pressure of the fluid. Non-supercritical fluid, for example liquid carbon dioxide, is fed into B1 *via* valve V3 to flush the precipitate from the filter F1 and form a suspension or slurry in the liquified fluid. This slurry is then fed into collection vessel B3 via valve V5, which is maintained at the desired operating temperature using heater H3. B3 is then depressurised *via* valve V10 and the solid product removed from filter F3 *via* an access port in B3. In the case where carbon dioxide is used, the operating pressure of the transfer step is *circa* 40 bar.

For the avoidance of doubt, it should be noted that the filters used in the apparatus, for example F1 and F3, must be configured to enable flushing of the product from the filter. An example of the flushing of the product from a filter is back-flushing. Suitable filters for use in the apparatus of the invention are, but not limited to, custom built radial filters. Other examples of suitable filter types are candle and coupon.

Suitably, the internal surface of the particle formation vessel(s) leading to the discharge point is of a suitable shape to facilitate discharge of the product, for example a conical shape.

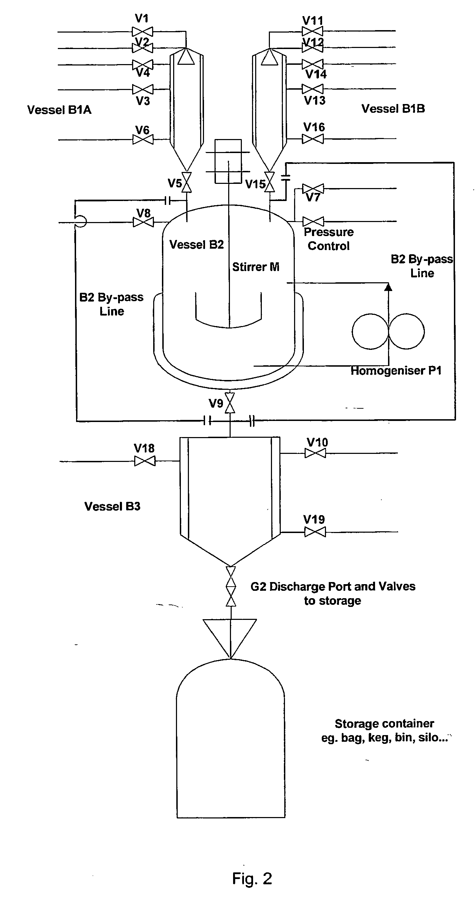

Fig. 2 shows a preferred embodiment of the invention. With reference to Fig. 2, there follows a detailed description of the operation of a preferred embodiment of the invention.

Vessels B1A and B1B are the particle formation vessels. During operation, these vessels operate alternately using an arrangement of switching valves i.e. one of the two particle formation vessels is being used for the particle formation whilst the product is being recovered from the other particle formation vessel.

Vessel B2 is a homogenisation vessel which is fitted with a stirrer (M) and a homogenisation loop running via a rotor-stator homogeniser P1. This vessel can be used to blend the batches of product from the formation vessels B1A and B1B. The homogenisation vessel may also optionally be used to blend any desired additives with the product. The use of this homogenisation step is optional and if preferred, the product from the particle formation steps may be directly transferred to B3 by appropriate arrangement of process flow lines. Vessel B3 is a collection vessel which is used to collect the batches of product produced in the formation vessels B1A and B1B. For convenience of removal of the product from B3, the working capacity of this vessel will be favourably selected to be similar to the capacity of the container in which the product is to be stored or transported.

Vessels B1A and B1B are brought to the desired operating temperature using jacket heaters and pressurised to the desired operating pressure by the inlet of supercritical fluid *via* a feed from valves V2 and V12.

Flow of the supercritical fluid is then started through B1A fed from V2 and exits via V6. It is recognised that further temperature control over the system can be achieved by controlling the fluid feed temperatures with suitable heat exchangers. Flow of the solution of the solid of interest is started and fed to B1A *via* valve V1. The precipitation of the product from its solution takes place in B1A and the precipitate is filtered *via* the radial filter in B1A. This process is continued until a desired amount of precipitate is collected in B1A.

B1A is then taken off-line by closure of valve V1. Flow of supercritical fluid may be maintained for a sufficient length of time so as to displace any residual organic solvent and prevent it associating with the product. Flow of SCF is then stopped and B1A partially depressurised *via* valve V6 to a pressure below the critical pressure of the SCF in use. Liquefied non-supercritical fluid is then fed into B1A *via* valve V3 to back-flush the product from the radial filter and form a slurry or suspension of the product in the liquefied non-supercritical fluid. This slurry or suspension is then either fed to the homogenisation vessel B2 where it accumulates or can be fed directly to the collection vessel B3 *via* a bottom outlet valve V5.

At the same time as B1A is taken off-line, flows of supercritical fluid and solution of the solid of interest to vessel B1 B are started through valves V12 and V11 respectively. As these two streams meet in vessel B1B, precipitation of the product commences and the precipitate is collected on the radial filter inside B1B. The mixture of SCF and solvent is continuously removed from vessel B1B *via* valve V16. This is continued until the desired amount of precipitate has been collected and at this point B1 B is brought off-line by closure of valve V11. Flow of supercritical fluid may be maintained for a sufficient length of time so as to displace any residual organic solvent and prevent it associating with the product. B1 B is then partially depressurised to below the critical pressure of the SCF in use and a feed of liquefied non-supercritical fluid is fed to the system via V13 to flush the radial filter and form a slurry or suspension of the product in the liquefied gas.

The product is then transported in the slurry or suspension either to the homogenisation vessel or to the bulk collection vessel B3.

This process of formation in one vessel B1A/B1B whilst the other B1B/B1A is being discharged, or switching process, is continued until the desired total quantity of material is formed and transferred to the homogenisation vessel or directly to B3.

As indicated above the entire production of product can then be blended and homogenised in vessel B2 as follows.

Liquefied non-supercritical fluid is be introduced into B2 until a desired level of liquid is reached. The stirrer, M, is then be started to create a homogeneous slurry or suspension of the solid product. The pump on the homogeniser loop P1 is then started and the slurry is cycled from the bottom of B2, through the homogeniser loop and into the top of B2. Once homogenisation is complete, V9 is opened and the slurry or suspension is transferred to B3, where the product is filtered. B3 is then depressurised *via* valve V10 (and/or V19) and the powder contents emptied into a storage keg through a bottom port (G2).

Typically, as in the case where carbon dioxide is used as the supercritical fluid, the particle formation vessels B1A and B1B will operate at pressures between 150 and 300 bar. The larger volume homogenisation step in B2 and filtration step in B3 will operate between 1 and 40 bar.

It is recognised that within the above description that throughout the process train employed the supercritical fluid employed to crystallise or precipitate the material was also used in its sub-critical form to transport the particulate product from the higher pressure formation environment through the process train to the lower pressure bulk product collection and final isolation stage. It is further recognised that it may be necessary and furthermore advantageous to employ a different fluid for the particle formation environment to that or those used within the bulk product blending/homogenisation and bulk product collection and final isolation stages. For example using carbon dioxide as the fluid for particle formation at supercritical pressures (typically, but not limited to, 100 - 300 bar), but using a fluid such as, but not limited to, 1,1,1,2-tetrafluoroethane, which has properties such that it liquefies at lower pressures and thus more convenient operating conditions for the bulk product blending/homogenisation and bulk product collection and final isolation stages may be employed. This has the advantage of further reducing the required operating and design pressures of the larger volume bulk product blending/homogenisation, bulk product collection, and final isolation stages of the process train and is recognised to possess advantages such as economic advantages.

As hereinbefore described, the benefits of the ability to transport a particulate product from a high pressure vessel such as B1A or B1B using a non-supercritical fluid, especially when incorporating a switching process, for example as depicted in Fig. 2 and described above, include the ability to achieve high product throughput while using smaller high-pressure vessels than would be required in a conventional batch process. Furthermore, the apparatus is easily adaptable to accommodate a plurality of particle formation vessels and/or collection vessels.

Throughout the description and the claims which follow, unless the context requires otherwise, the word 'comprise', and variations such as 'comprises' and 'comprising', will be understood to imply the inclusion of a stated integer or step or group of integers but not to the exclusion of any other integer or step or group of integers or steps.

The following Examples serve to illustrate the invention but do not limit it in any way.

### Examples

### Example 1 Description to demonstrate the use and operation of system with a SCF anti-solvent process as the particle formation step.

The following example will be used to illustrate the use and operation of the process outlined in Figure 2. The vessels B1A and B1B were of 0.5 litre volume. Vessels B2 and B3 were of 5 litres volume. All vessels in this example were custom-built by SITEC Eng. AG. Vessels B1A, B1B, B2 and B3 have conical bases to assist in the free transport of materials from their bases. Within vessels B1A, B1 B and B3 were 0.5 micron sintered stainless steel cylindrical (radial) filter elements which form a filter barrier between the internal components of the vessel and the fluid exit line(s). Vessels B1A and B1 B are heated to eg. 45°C using jacket heat exchangers. Vessel B2 and B3 were brought to a temperature of 5°C using jacket coolers. With valves V5 and V15 closed, valves V2 and V12 are opened and vessels were filled and pressurised with supercritical carbon dioxide to a pressure of 150 bar. This pressure is maintained by correct setting of the back pressure controllers V16 and V6. Vessels B2 and B3 are pressurised to 40 bar with carbon dioxide (gas-liquid mixture) via valves V8 and V18. Valve 12 was closed and flow of supercritical carbon dioxide through vessel B1A is started at a rate of 15kg/hr and at a temperature of eg. 45°C through valve V2, the vessel B1A, and exits via valve V6. Flow of a solution of eg. paracetamol in ethanol (100mg/ml concentration) is pumped at a rate of 5mlmin⁻¹ through valve V1 to a spray head located inside the lid of vessel B1A. As this solution flow contacts the supercritical carbon dioxide anti-solvent, precipitation of the solute e.g. paracetamol as a finely divided powder commences. This flow of solution and supercritical fluid through B1A is continued until *circa* 60g of paracetemol is formed in B1A. At this point 10 ml of ethanol is fed in *via* valve V1 to displace solution from the feed line. Valve V1 is then closed and flow of liquid solvents to vessel B1A was stopped. The flow of supercritical carbon dioxide is continued at a rate of 15kg/hr for a further 10 minutes and then stopped by closure of V2. The pressure inside B1A is lowered to 40 bar by adjustment of valve V6. Valves V5 and V3 are opened and liquid carbon dioxide is fed through the filter in B1A, washing off the filter cake and carrying the product in suspension into vessel B2 via valve V5. The pressure within B2 is controlled by a suitable pressure regulator. At this point, valve V5 is closed and V2 opened and the pressure in B1A returned to 150 bar as before. Simultaneously, valve V12 was opened and the flow of supercritical carbon dioxide through B1 B started at the same conditions used above. This is the start of the switching step which brings particle formation vessel B1B online as B1A is brought offline. Flow of the paracetamol solution to vessel B1B is then started using the same conditions as used for B1A. This flow of solution is continued until *circa* 60g of paracetamol powder is formed in B1B. At this point 10 ml of ethanol is fed in *via* valve V11 to displace solution from the feed line. Valve V11 is then closed and flow of liquid solvents to vessel B1B stopped. The flow of supercritical carbon dioxide is continued at a rate of 15kg/hr for a further 10 minutes and then stopped by closure of V12. The pressure inside B1 B is lowered to 40 bar by adjustment of valve V16. Valves V15 and V13 were opened and liquid carbon dioxide was fed backwards through the filter in B1B, washing off the filter cake and carries the product in suspension into vessel B2 via valve V15. The pressure within B2 is controlled by a suitable pressure regulator. At this point, valve V15 is closed and V12 opened and the pressure in B1B returned to 150 bar as before. At this point B1A was brought back online and the process of switching particle formation and removal between the respective vessels is continued until eg. 1kg of product is collected in B2. Throughout the formation and transfer process, the stirrer, homogeniser and liquid level in B2 are controlled. Once homogenisation is complete, valve V9 was opened and the suspension drained into B3. The product is then isolated as a dry solid by isolating B3 (closure of valve V9 and the port in the base), and valve V19 is opened to allow depressurisation of B3. Once depressurised the port in the base of B3 is connected to a collection keg and the powder product discharged ready for storage or use.

### Example 2 Demonstration of the inclusion of a homogenisation and blending step in conjunction with the powder transport steps.

The following example will be used to demonstrate the application and use of the equipment outlined in Figure 2 in terms of the use of the homogenisation step carried out in a liquefied gas included with transport steps through the system. Furthermore it will be recognised that within this example, 1,1,1,2-tetrafluoroethane is used as the working fluid at a pressure of less than 10 bar, which will be obvious to those familiar with such processes that this offers considerable operational and cost benefits, especially in cases where for instance the steps required to form the particulates required the use of a high pressure process, such as a supercritical fluid process.

### Example 2a

49.9808g of lactose and 0.01954g of fluticasone propionate were charged to vessel B1A. Giving a nominal 0.04wt% fluticasone propionate concentration in the lactose. Vessel B1A was connected to vessel B2 via valve V5 and 1 kg of 1,1,1,2-tetrafluoroethane at 7bar and 20C was introduced into the system via valve V2 and allowed to flow through vessel B1A to B2. This flushed the lactose and fluticasone into the vessel B2 in the form of a suspension. Valve V5 was then closed and the stirrer (M) and Homogeniser (P1) started at 200RPM and 1700RPM respectively. This homogenisation and blending was continued for 20 minutes at which point valve V9 was opened and the resulting suspension was drained into vessel B3. Valve V5 and V19 were then opened a further 0.5kg of 1,1,1,2-tetrafluoroethane was passed through B1A, B2 and into B3 to wash and flush out any remaining powder in vessel B2. Valve V9 was then closed and compressed air was introduced into B3 via valve V8 (at a static pressure of 8 bar) to displace and expel the liquid 1,1,1,2-tetrafluoroethane out of the bottom of vessel B3 via valve V19. After 2 minutes of passing compressed air through the system, V8 was shut and B3 was allowed to slowly depressurise through valve V19. The powder product was then discharged into a bag via the discharge port G2 in vessel B3. Samples of the powder were taken and these were analysed by HPLC to test for content uniformity of the fluticasone propionate in the lactose.

### Example 2b

49.8107g of lactose and 0.2023g of fluticasone propionate were charged to vessel B1A. Giving a nominal 0.4wt% fluticasone propionate concentration in the lastose. Vessel B1A was connected to vessel B2 via valve V5 and 1 kg of 1,1,1,2-tetrafluoroethane at 7bar and 20C was introduced into the system via valve V2 and allowed to flow through vessel B1A to B2. This flushed the lactose and fluticasone into the vessel B2 in the form of a suspension. Valve V5 was then closed and the stirrer (M) and Homogeniser (P1) started at 200RPM and 1700RPM respectively. This homogenisation and blending was continued for 20 minutes at which point valve V9 was opened and the resulting suspension was drained into vessel B3. Valve V5 and V19 were then opened a further 0.5kg of 1,1,1,2-tetrafluoroethane was passed through B1A, B2 and into B3 to wash and flush out any remaining powder in vessel B2. Valve V9 was then closed and compressed air was introduced into B3 via valve V8 (at a static pressure of 8 bar) to displace and expel the liquid 1,1,1,2-tetrafluoroethane out of the bottom of vessel B3 via valve V19. After 2 minutes of passing compressed air through the system, V8 was shut and B3 was allowed to slowly depressurise through valve V19. The powder product was then discharged into a bag via the discharge port G2 in vessel B3. Samples of the powder were taken and these were analysed by HPLC to test for content uniformity of the fluticasone propionate in the lactose.

### Example 2c

47.9907g of lactose and 1.9978g of fluticasone propionate were charged to vessel B1A. Giving a nominal 4wt% fluticasone propionate concentration in the lastose. Vessel B1A was connected to vessel B2 via valve V5 and 1 kg of 1,1,1,2-tetrafluoroethane at 7bar and 20C was introduced into the system via valve V2 and allowed to flow through vessel B1A to B2. This flushed the lactose and fluticasone into the vessel B2 in the form of a suspension. Valve V5 was then closed and the stirrer (M) and Homogeniser (P1) started at 200RPM and 1700RPM respectively. This homogenisation and blending was continued for 20 minutes at which point valve V9 was opened and the resulting suspension was drained into vessel B3. Valve V5 and V19 were then opened a further 0.5kg of 1,1,1,2-tetrafluoroethane was passed through B1A, B2 and into B3 to wash and flush out any remaining powder in vessel B2. Valve V9 was then closed and compressed air was introduced into B3 via valve V8 (at a static pressure of 8 bar) to displace and expel the liquid 1,1,1,2-tetrafluoroethane out of the bottom of vessel B3 via valve V19. After 2 minutes of passing compressed air through the system, V8 was shut and B3 was allowed to slowly depressurise through valve V19. The powder product was then discharged into a bag via the discharge port G2 in vessel B3. Samples of the powder were taken and these were analysed by HPLC to test for content uniformity of the fluticasone propionate in the lactose.

The results for the content uniformity of the materials produced above in Examples 2a, 2b and 2c were as shown in Table 2.

**Table 2**

| Example Reference | Minimum fluticasone Concentration | Mean fluticasone concentration | Maximum fluticasone concentration | Relative Standard Deviation |
|---|---|---|---|---|
| | wt% fluticasone in total blend | | | |
| 2a | 0.0116 | 0.0123 | 0.0133 | 4.54 |
| 2b | 0.305 | 0.331 | 0.351 | 4.34 |
| 2c | 3.21 | 3.51 | 3.78 | 5.55 |
| Control Blend | 0.33 | 0.35 | 0.36 | 2.95 |
| | | | | |

| | | | | |
|---|---|---|---|---|
| Note: For purposes of clarity, "control blend" data refers specifically to data obtained from a high shear conventional dry powder blend which has been optimised at a magnitude of scale comparable with that of the Examples. | | | | |

### Example 2d

96.6 grams of lactose monohydrate and 0.4 grams of Compound A were charged to vessel B1A. This gave a nominal 0.4wt% Compound A concentration in the lactose. Vessel B1A was connected to vessel B2 via valve V5 and 1.4kg of 1,1,1,2-tetrafluoroethane at 6bar and 20°C was introduced into the system via valve V2 and allowed to flow through vessel B1A to B2. This flushed the lactose and Compound A into the vessel B2 in the form of a suspension. Valve V5 was then closed and the stirrer (M) and Homogeniser (P1) started at 160RPM and 3600RPM respectively. This homogenisation and blending was continued for 30 minutes at which point valve V9 was opened and the resulting suspension was drained into vessel B3. Valve V9 was then closed and compressed air was introduced into B3 via valve V8 (at a static pressure of 8 bar) to displace and expel the liquid 1,1,1,2-tetrafluoroethane out of the bottom of vessel B3 via valve V19. After approximately1 minute of passing compressed air through the system, V8 was shut and B3 was isolated and allowed to slowly depressurise through valve V19. The powder product was then removed and recovered. 10 samples of the powder blend were taken and tested by HPLC analysis to ascertain content uniformity to test the quality of the blending process. The results of which are shown in Table 2a below.

**Table 2a**

| Sample Number | Compound A concentration % w/w | % Nominal target concentration (0.4%) |
|---|---|---|
| | | |
| 1 | 0.39 | 102.03 |
| 2 | 0.39 | 100.82 |
| 3 | 0.37 | 97.19 |
| 4 | 0.38 | 99.97 |
| 5 | 0.38 | 97.70 |
| 6 | 0.39 | 100.42 |
| 7 | 0.36 | 93.86 |
| 8 | 0.37 | 97.12 |
| 9 | 0.37 | 95.76 |
| 10 | 0.39 | 100.76 |
| Mean Value | 0.38 | 98.53 |
| % Relative Standard Deviation | 2.73 | |

### Example 3 Semi-continuous operation of a supercritical fluid anti-solvent process with semi-continuous powder transport and recovery.

Example 3 illustrates the semi-continuous use and operation of the process train outlined in figure 2. Note, vessel B2 has been by-passed using the bypass lines to connect valves V5 and V15 to vessel B3.

The vessels B1A and B1B are 0.5 litre volume. Vessel B3 is 5 litres volume. All vessels in this example were custom-built by SITEC Eng. AG. Vessels B1A, B1B and B3 have conical bases to assist in the free transport of materials from their bases. Within vessels B1A, B1B and B3 were 0.5 micron sintered stainless steel cylindrical (radial) filter elements which form a filter barrier between the internal components of the vessel and the fluid exit line(s). The temperature of vessels B1A and B1B are controlled using jacket heat exchangers which are fed by a heat transfer fluid. The temperature of vessel B3 was maintained at 5°C using jacket coolers. With valves V5 and V15 closed, valves V2 and V12 were opened and vessels were filled and pressurised with supercritical carbon dioxide to a pressure of 150 bar. This pressure was maintained by correct setting of the back pressure controllers V16 and V6. Vessel B3 was pressurised to 60 bar with liquid carbon dioxide via V18. Valve 12 was closed and flow of supercritical carbon dioxide through vessel B1A was started at a rate of 12kg/hr and at a temperature of 45°C through valve V2, the vessel B1A, and exits via valve V6. Flow of a 4wt% solution of paracetamol in 90:10w/w methyl acetate:methanol was pumped at a rate of 5mlmin⁻¹ through valve V1 to a 150micron orifice nozzle located inside the lid of vessel B1A. As this solution flow contacts the supercritical carbon dioxide anti-solvent, precipitation of the paracetamol as a finely divided powder commences. This flow of solution and supercritical fluid through B1A was continued for 4 hours. At this point a flow 90:10w/w methyl acetate:methanol was fed in *via* valve V1 to displace solution from the feed line. This was carried out for 10 minutes at a rate of 5ml/min. Valve V1 was then closed and flow of liquid solvents to vessel B1A was stopped. The flow of supercritical carbon dioxide was continued at a rate of 12kg/hr for a further 30 minutes and was then stopped by closure of V2. The pressure inside B1A was lowered to 60 bar by adjustment of valve V6. Valves V5 and V3 were opened and liquid carbon dioxide was fed through the filter in B1A at a rate of 12kg/hr, washing off the filter cake and carrying the product in suspension into vessel B3 via valve V5. The pressure within B3 was controlled by adjustment of V19. At this point, valve V5 was closed and V2 opened and the pressure in B1A returned to 150 bar as before. Simultaneously, valve V12 was opened and the flow of supercritical carbon dioxide through B1B was started at the same conditions used above. This was the start of the switching step which brings particle formation vessel B1B online as B1A was brought offline. Flow of the paracetamol solution to vessel B1B was then started using the same conditions as was used for B1A. This flow of solution was continued for 4 hours to form paracetamol powder by an SCF anti-solvent process. After the 4 hours of particle formation, the solution flow was stopped. At this point a flow of 90:10 w/w methyl acetate:methanol was fed in *via* valve V11 to displace solution from the feed line. This flow was continued for 10 minutes at a rate of 5ml/min. Valve V11 was then closed and flow of liquid solvents to vessel B1B was stopped. The flow of supercritical carbon dioxide was continued at a rate of 12kg/hr for a further 30 minutes and was then stopped by closure of V12. The pressure inside B1B was lowered to 60 bar by adjustment of valve V16. Valves V15 and V13 were opened and liquid carbon dioxide was fed backwards through the filter in B1B, washing off the filter cake and carries the product in suspension into vessel B3 via valve V15. The pressure within B3 was controlled by a suitable adjustment of valve V19. At this point, valve V15 was closed and V12 opened and the pressure in B1B returned to 150 bar as before. At this point B1A was brought back online and the process of switching particle formation and removal between the respective vessels was continued for a total of 6 cycles (i.e. 3 cycles in each particle formation vessel). The product was then isolated as a dry solid by isolating B3 (closure of valve V9 and the port in the base), and valve V19 was opened to allow depressurisation of B3. Once depressurised the port in the base of B3 was connected to a collection keg (not shown) and the powder product was discharged ready for storage or use.

This above total process of 6 particle formation cycles with transfer of the product to B3 after each cycle followed by discharge of the product from B3 after 6 particle formation cycles was continued over the course of 5 days fully continuous 24 hour operation to demonstrate the full operation of the particle formation, transport and discharge steps in fully semi-continuous mode.

### Example 4 Further example of the formation of a particuate product using CO₂ as the supercritical fluid for particle formation and liquefied CO₂ as the transport media.

Note, in this specific Example, vessel B2 has been by-passed using the bypass lines to connect valves V5 and V15 to vessel B3. However it will be appreciated that in conjunction with Examples 2a to 2d, that it is equally possible to carry out the particle formation step and transport the materials into vessel B2 for blending with another component should this be required. Furthermore within the following Example, only a single particle formation and transport step has been conducted, however it will be appreciated by the detail given in Example 3 that it is possible to carry out multiple formation and transport cycles.

With valve V5 and V15 closed, supercritical carbon dioxide was fed into vessel B1A via V2 and the vessel brought to an operating pressure of 150 bar at 55C. With a flow of carbon dioxide maintained at 15kg/hr (at 150 bar and 55C ) through valve V2 into vessel B1A, through the filter insert in B1A and leaving through valve V6, a solution of ropinirole hydrochloride (33 mg/ml in methanol) at 5ml/min was fed to the system via valve V1 to a union tee which connects the lines coming from valves V1 and V2 with the internals of vessel B1A. As the solution and stream of supercritical carbon dioxide meet, precipitation of the ropinirole hydrochloride takes place and the precipitate was retained on a filter element inside vessel B1A. The CO₂-methanol mixture leaves vessel B1A via valve V6. After a sufficient quantity of ropinirole hydrochloride solution was fed to the vessel (giving a corresponding quantity of precipitate, in this case 1200 ml solution and approximately 40 grams of precipitate) solution flow was stopped. The flow of CO₂ at 150 bar, 55C and 15kg/hr was maintained for a further 30 minutes to displace any residual methanol from the system. Valve V2 and V1 were then closed and the pressure inside vessel B1A was lowered to 60 bar using valve V6. Valve V6 was then closed. Valve V5 was then opened (to allow connection of vessel B1A with vessel B3) and valves V3 and V19 opened. A flow of liquid carbon dioxide at 60 bar and 20°C and 15kg/hr was fed into vessel B1A via valve V3, through the filter inside B1A (in the opposite direction of flow during the precipitation step in order to back flush precipitate from the filter) out of vessel B1A via valve V5, into vessel B3, across the filter located inside B3 and out of B3 via valve V19. This is in order to remove the precipitate from vessel B1A and transport and collect it in vessel B3. After 30 minutes of flushing in this manner, vessel B3 was isolated from B1A by closing valve V5 and the liquid carbon dioxide was drained from vessel B3 via valve V19 and vessel V3 allowed to depressurise to ambient pressure. Valves G2 were then opened to allow the powder product to be discharged from B3 and collected into the storage container.

### Example 5 Demonstration of the formation of product using CO2 as the supercritical fluid for particle formation and using a lower vapour pressure liquefied gas as the transport media.

It has been discussed earlier that further benefits of the present invention may be brought about if a liquefied gas with a lower vapour pressure is employed as the transport media. Clearly this will reduce the required pressure rating of both the blending and homogenisation stages as well as the bulk collector (B3 in Figure 2) used prior to final discharge into an ambient pressure collector or storage keg.

Note, in this specific example, vessel B2 has been by-passed using the bypass lines to connect valves V5 and V15 to vessel B3. However it will be appreciated that in conjunction with Examples 2a to 2d, that it is equally possible to carry out the particle formation step and transport the materials into vessel B2 for blending with another component should this be required. Furthermore within the following Example, only a single particle formation and transport step has been conducted, however it will be appreciated by the detail given in Example 3 that it is possible to carry out multiple formation and transport cycles.

With valve V5 and V15 closed, supercritical carbon dioxide was fed into vessel B1A via V2 and the vessel brought to an operating pressure of 150 bar at 55C. With a flow of carbon dioxide maintained at 15kg/hr (at 150 bar and 55C ) through valve V2 into vessel B1A, through the filter insert in B1A and leaving through valve V6, a solution of ropinirole hydrochloride (33 mg/ml in methanol) at 5ml/min was fed to the system via valve V1 to a union tee which connects the lines coming from valves V1 and V2 with the internals of vessel B1A. As the solution and stream of supercritical carbon dioxide meet, precipitation of the ropinirole hydrochloride takes place and the precipitate was retained on a filter element inside vessel B1A. The CO₂-methano mixture leaves vessel B1A via valve V6. After a sufficient quantity of ropinirole hydrochloride solution was fed to the vessel (giving a corresponding quantity of precipitate, in this case 1200 ml solution and approximately 40 grams of precipitate) solution flow was stopped. The flow of CO₂ at 150 bar, 55C and 15kg/hr was maintained for a further 30 minutes to displace any residual methanol from the system. Valve V2 and V1 were then closed and the remaining CO₂ discharged via valve V6. Valve V5 was then opened (to allow connection of vessel B1A with vessel B3) and valves V3 and V19 opened. A flow of 1,1,1,2 tetrafluoroethane at 10 bar, 20C and 10kg/hr was fed into vessel B1A via valve V3, through the filter inside B1A (in the opposite direction of flow in order to back flush precipitate from the filter) out of vessel B1A via valve V5, into vessel B3, across the filter located inside B3 and out of B3 via valve V19. This is in order to remove the precipitate from vessel B1A and collect it in vessel B3.

After 30 minutes of flushing in this manner, vessel B3 was isolated from B1A by closing valve V5 and the liquid 1,1,1,2-tetrafluoroethane was drained from vessel B3 via valve V19 and vessel V3 allowed to depressurise to ambient pressure. Valves G2 were then opened to allow the powder product to be discharged from B3 and collected into the storage container.

## Claims

1. A process for the isolation of a particulate pharmaceutical product from a supercritical process, which process comprises the isolation of the pharmaceutical product as a suspension in a non-supercritical fluid, wherein the pharmaceutical product is a pharmaceutically-active compound and/or pharmaceutical excipient and wherein the fluid used in the process is selected from nitrous oxide, sulphur hexafluoride, xenon, ethylene, chlorotrifluoromethane, ethane, trifluoromethane, 1,1,1,2-tetrafluoroethane and carbon dioxide.

2. A process according to claim 1 wherein the supercritical fluid contains one or more modifiers.

3. A process according to claim 1 or claim 2 wherein the pharmaceutical product consists of multiple components.

4. A process according to any one of claims 1 to 3 comprising the further step of volatilisation of the non-supercritical fluid,

## Patentansprüche

1. Verfahren zur Isolierung eines partikulären pharmazeutischen Produkts aus einem superkritischen Verfahren, wobei das Verfahren die Isolierung des pharmazeutischen Produkts als eine Suspension in einem nicht-superkritischen Fluid umfaßt, worin das pharmazeutische Produkt eine pharmazeutisch wirksame Verbindung und/oder ein pharmazeutischer Exzipient ist, und worin das im Verfahren verwendete Fluid ausgewählt ist aus Distickstoffoxid, Schwefelhexafluorid, Xenon, Ethylen, Chlortrifluormethan, Ethan, Trifluormethan, 1,1,1,2-Tetrafluorethan und Kohlendioxid.

2. Verfahren gemäß Anspruch 1, worin das superkritische Fluid einen oder mehrere Modifikatoren enthält.

3. Verfahren gemäß Anspruch 1 oder Anspruch 2, worin das pharmazeutische Produkt aus mehreren Komponenten besteht.

4. Verfahren gemäß einem der Ansprüche 1 bis 3, das ferner den Schritt der Verflüchtigung des nicht-superkritischen Fluids umfaßt.

## Revendications

1. Procédé pour l'isolation d'un produit pharmaceutique particulaire à partir d'un procédé supercritique, lequel procédé comprend l'isolation du produit pharmaceutique sous la forme d'une suspension dans un fluide non supercritique, dans lequel le produit pharmaceutique est un composé ayant une activité pharmaceutique et/ou un excipient pharmaceutique, et dans lequel le fluide utilisé dans le procédé est choisi parmi l'oxyde nitreux, l'hexafluorure de soufre, le xénon, l'éthylène, le chlorotrifluorométhane, l'éthane, le trifluorométhane, le 1,1,1,2-tétrafluoroéthane et le dioxyde de carbone.

2. Procédé selon la revendication 1, dans lequel le fluide supercritique contient un ou plusieurs agents modificateurs.

3. Procédé selon la revendication 1 ou la revendication 2, dans lequel le produit pharmaceutique est constitué de composants multiples.

4. Procédé selon l'une quelconque des revendications 1 à 3, comprenant l'étape supplémentaire de volatilisation du fluide non supercritique.
